Europäisches Patentamt

European Patent Office

Office européen des brevets

(11) Numéro de publication: **0 242 300 B1**

# FASCICULE DE BREVET EUROPEEN

(45) Date de publication de fascicule du brevet:
**21.08.91**

(51) Int. Cl.⁵: **G01N 33/576**

(21) Numéro de dépôt: **87400863.4**

(22) Date de dépôt: **15.04.87**

(54) **Nouveau réactif pour la détection d'hépatites virales non-a non-b et procédé de diagnostic de telles hépatites par voie immunoenzymatique.**

(30) Priorité: **16.04.86 FR 8605437**

(43) Date de publication de la demande:
**21.10.87 Bulletin 87/43**

(45) Mention de la délivrance du brevet:
**21.08.91 Bulletin 91/34**

(84) Etats contractants désignés:
**AT BE CH DE ES FR GB GR IT LI LU NL SE**

(56) Documents cités:
**EP-A- 0 066 296      EP-A- 0 068 465**
**EP-A- 0 124 896      EP-A- 0 154 392**
**WO-A-80/02598       WO-A-82/00205**
**FR-A- 2 502 154**

(73) Titulaire: **INSTITUT PASTEUR**
**28, rue du Docteur Roux**
**F-75724 Paris Cédex 15(FR)**

(72) Inventeur: **Pillot, Jacques**
**14 Chemin de Moulon**
**F-91190 Gif-sur-Yvette(FR)**

(74) Mandataire: **Ores, Irène et al**
**CABINET ORES 6, Avenue de Messine**
**F-75008 Paris(FR)**

Rank Xerox (UK) Business Services

## Description

La présente invention est relative à un nouveau réactif pour la détection d'hépatites virales non-A non-B et à un procédé de diagnostic de telles hépatites par voie immunoenzymatique.

Les hépatites non-A non-B sont définies comme étant des hépatites infectieuses virales qui ne peuvent être attribuées ni au virus de l'hépatite A (HAV), ni au virus de l'hépatite B (HBV), ni à d'autres virus hépatotropes humains connus tel que le Cytomégalovirus, le virus d'Epstein-Barr ou le virus de la fièvre jaune. Les hépatites non-A non-B semblent être un groupe d'affections dues à plusieurs virus différents.

En Europe de l'Ouest et en Amérique du Nord, 95% des hépatites post-transfusionnelles sont dues à des agents infectieux qui se transmettent par voie parentérale, de la même façon que l'hépatite B, comme le rapportent E.TABOR [Lancet, 1985, i : 743-745] et D.G. Wong et al [Lancet, 1980, ii : 876-879].

Des données épidémiologiques récentes ont mis en évidence l'existence de formes de NANBH qui se transmettent comme l'hépatite A, c'est-à-dire par voie fécale-orale et pratiquement jamais par transfusion. Plusieurs épidémies de ce type ont été rapportées (M.S., Balayan et al; Intervirology, 1983, 20 : 23-31 - E.H., Belabbes et al; J. Med. Virol., 1985, 16 : 257-263 - M.A., Kane et al; JAMA 1984, 252 : 3140-3145 - M.S., Khuroo; Am. J. Med., 1980, 68 : 818-824). Ces épidémies sont d'origine hydrique, avec un mode de transmission fécal-oral et des particules virales de 27 nm ont été observées par microscope électronique d'échantillons obtenus à partir de fèces de malades (M.S., Balayan et al; Intervirology, 1983, 20 : 23-31 - M.A., Kane et al; JAMA, 1984, 252 : 3140-3145).

Comme on le sait, les virus d'hépatites non-A non-B sont à l'origine d'affections hépatiques graves telles que cirrhose du foie, carcinome hépatocellulaire non établi et analogues, en sorte que l'identification de ces virus et la détection du système antigène-anticorps associé à ces virus est de la plus haute importance pour la réalisation d'un diagnostic précoce et l'instauration rapide d'une prophylaxie appropriée.

Les réactifs et les méthodes de diagnostic proposés jusqu'à présent concernent essentiellement la détection de l'antigène associé à un virus d'hépatite non-A non-B transmis à l'occasion de transfusions sanguines.

C'est ainsi que SHIRASHI et Al. [THE LANCET, 21 Octobre 1978, p. 853-856] ont détecté chez des êtres humains atteints d'hépatite non-A non-B consécutive à une transfusion, un système antigène-anticorps, par contre-électrophorèse, et que la demande internationale PCT publiée sous le numéro 80/02598 déposée le 20 Mai 1980 en revendiquant une priorité US n° 040921 du 21 MAI 1979, a fait application des travaux de SHIRASHI et Al., en revendiquant un test immunologique de détection d'un antigène associé à une hépatite non-A non-B (NANBH), dans lequel on fait réagir un échantillon de sérum d'un mammifère supposé atteint, avec un anticorps provenant du sérum d'un mammifère donneur que l'on sait porteur d'une infection par NANBH et l'on détecte la présence de l'antigène de NANBH soit indirectement par l'intermédiaire de l'immunoprécipitine formée par contre-électrophorèse, soit par des méthodes directes telles que radioimmunoessai, diffusion en gel de gélose, hémagglutination passive, agglutination sur latex, fixation du complément ou test ELISA.

Par ailleurs, la Demande internationale PCT n° WO 82/00205, décrit une particule virale de 50 à 60 nm de diamètre qui possède un noyau d'environ 40 nm de diamètre et qui serait l'agent causal d'une hépatite non-A non-B. Quatre échantillons contenant cette particule virale ont été déposés à la "American Type Culture Collection" sous les numéros VR-2011, VR-2012, VR-2013 et VR-2014.

Les particules décrites peuvent être radiomarquées pour être utilisées dans un test de détection.

D'autres Auteurs ont cherché à isoler l'antigène associé à la NANBH et à utiliser cet antigène isolé pour préparer un réactif de diagnostic des infections provoquées par le virus de l'hépatite virale NANB. En particulier le Brevet Français TREPO 81 06385 du 30 Mars 1981 isole un nouvel antigène de NANBH par ultracentrifugation de sérums ou d'extraits de foie, traitement du culot de centrifugation par un détergent non ionique, puis fractionnement pour isoler les fractions contenant de l'antigène NANBc purifié (éventuellement suivi d'un traitement par un détergent ionique pour isoler l'AgNANBe) ou, selon une variante ce Brevet Français propose, après avoir éliminé, comme ci-dessus, les particules virales éventuellement présentes, par ultracentrifugation, d'éliminer les gammaglobulines, de concentrer l'AgNANBe par précipitation, de chromatographier les fractions concentrées contenant l'AgNANBe sur un support sur lequel est fixée de l'héparine, et de recueillir l'AgNANBe dans les éluats. Les réactifs de diagnostic conformes au Brevet Français TREPO comprennent un support solide sur lequel est fixé au moins un antigène NANBc, NANBe ou NANBs et/ou au moins un anticorps spécifique anti-NANBc, anti-NANBe ou anti-NANBs; dans le cas particulier du réactif de diagnostic dont le support porte des anticorps anti-(gammaglobulines humaines), l'antigène est séparé du support et ne peut éventuellement être fixé que par l'intermédiaire des anticorps correspondants du sérum à tester, s'ils sont présents.

De même, la Demande de Brevet Européen EISAI publiée sous le numéro 66 296 et déposée le 2 Juin 1982 en revendiquant une priorité Japonaise du 2 Juin 1981, décrit l'isolement d'un antigène associé à l'hépatite NANB, par purification à partir de foie prélevé par autopsie de malades décédés d'hépatite NANB, par des méthodes classiques de fractionnement de protéines, y compris l'ultracentrifugation, et définit cet antigène par ses propriétés physicochimiques; l'antigène selon cette demande de brevet Européen est conjugué à une substance qui peut consister en des particules de support convenant à l'hémagglutination passive telles que les érythrocytes de mouton, des isotopes traceurs pour les radioimmunoessais ou des enzymes convenant à la réalisation du test ELISA.

Il ne semble cependant pas que l'on ait proposé à ce jour des moyens permettant l'identification d'un système antigène-anticorps associé à une forme épidémique d'hépatite NANB.

La présente invention a pour but de pourvoir à un réactif propre à permettre la détection d'hépatites virales NANB épidémiques et à un procédé de diagnostic mettant en oeuvre ce réactif.

La présente invention a pour objet un anticorps anti-NANBH constitué par des IgM isolées de sérums de singes infectés artificiellement par des extraits de fèces de patients connus pour être atteints d'hépatite NANB épidémique.

La présente invention a également pour objet un procédé d'isolement d'un anticorps anti-NANBH à partir de sérums de singes artificiellement infectés par ingestion d'extraits de fèces de patients atteints d'hépatite NANB épidémique, caractérisé en ce que lesdits sérums sont traités par chromatographie sur colonne de gel de dextrane dans du tampon Tris-NaCl 0,1 M, pH 8,0, pour isoler des IgM anti-NANBH.

Selon un mode de mise en oeuvre du procédé d'isolement des IgM anti-NANBH conforme à la présente invention, les sérums de singes artificiellement infectés mis en oeuvre sont des sérums prélevés à partir du 27ème jour après l'inoculation.

La présente invention a en outre pour objet un réactif propre à permettre la détection d'hépatites virales NANB épidémiques, qui est caractérisé en ce qu'il comprend des IgM anti-NANBH fixées sur des supports solides et notamment sur des plaques de PVC.

La présente invention a également pour objet un procédé de préparation d'un réactif propre à permettre la détection d'hépatites virales NANB épidémiques, qui est caractérisé en ce que les IgM anti-NANBH isolées conformément à l'invention sont fixées sur des supports solides et notamment sur des plaques en PVC, par contact des IgM à des concentrations de l'ordre de 50 $\mu$g de protéines par ml de PBS, avec lesdits supports solides, pendant 18 à 24 heures à 4°C.

Selon un mode de mise en oeuvre du procédé de préparation d'un réactif de détection d'hépatites virales NANB épidémiques conforme à l'invention, les plaques de PVC revêtues d'IgM anti-NANBH sont ensuite enduites d'une couche de PBS contenant 0,1% de BSA et de 0,1% de "Tween®20".

La présente invention a en outre pour objet une méthode de diagnostic d'hépatites virales NANB épidémiques, caractérisée en ce que le réactif de détection constitué par les plaques enduites d'IgM est incubé avec des extraits de fèces pendant 1 heure à 37°C, puis avec des fractions d'IgG purifiées à partir de sérums de patients convalescents de NANBH épidémique et marquées par une enzyme, en présence d'un substrat révélateur approprié.

Selon un mode de mise en oeuvre préféré de la méthode de diagnostic conforme à l'invention, les fractions d'IgG sont marquées à la $\beta$-galactosidase et le substrat révélateur approprié est de préférence constitué par l'orthonitrophényl-$\beta$-D-galactopyranoside.

Outre les dispositions qui précèdent, l'invention comprend encore d'autres dispositions, qui ressortiront de la description qui va suivre.

L'invention sera mieux comprise à l'aide du complément de description qui va suivre, qui se réfère à un exemple de mise en oeuvre du procédé objet de la présente invention, donné uniquement à titre d'illustration.

EXEMPLE

1 - Détermination du caractère NANB du virus d'hépatite utilisé comme matière première Des échantillons de sérums ont été prélevés chez 37 patients présumés atteints d'une hépatite virale NANB aigüe épidémique, dans la zone de TORTIYA, en COTE D'IVOIRE, au cours de la première semaine de la jaunisse.

Ces échantillons ont été soumis aux tests de détection suivants dans le but d'établir avec certitude le caractère NANB du virus :
- tests de détection du virus de l'hépatite A et du virus de l'hépatite B à l'aide des kits ELISA disponibles dans le commerce en provenance de ABBOTT Laboratories (CHICAGO, ILLINOIS), à savoir :

. test HAVAB pour la détection des IgM ou des IgG anti-virus A,

. test "AUSZYME" pour la détection d'AgHBs,

. test HBe-EIA pour la détection d'AgHBe,

. test "CORZYME" pour le dosage des anticorps totaux dirigés contre la capside du virus de l'hépatite B,

. test "CORZYME M" pour le dosage des IgM spécifiques dirigées contre la capside du virus de l'hépatite B,

. tests immunoenzymatiques selon SCHMITZ et Al [J. Gen. Virol., 1980, 50 : 59-68 ] pour le dosage des anticorps dirigés contre le CMV,

. dosage des IgM dirigées contre le virus d'Epstein-Barr par immunofluorescence selon GARDNER et Al. dans "Rapid virus diagnosis : application of immunofluorescence", BUTTER-WORTHS Ed. LONDRES, 1980, p. 276-278,

. dosage des IgM anti-fièvre jaune par immunocapture selon la technique de LHUILLIER et Al. Ann. Virol. (Inst. Pasteur), 1983, 134E, p. 349-359.

Les 37 échantillons de sérums testés provenant de patients présumés atteints de NANBH comprenaient tous des anticorps anti-VHA, mais aucun ne contenait d'anticorps spécifiques anti-VHA de la classe des IgM.

32 sérums sur 37 possédaient des anticorps anti-HBc (anticorps dirigés contre l'antigène de capside du virus de l'hépatite B), mais aucun n'avait d'anticorps spécifiques anti-HBc de la classe des immunoglobulines IgM.

Deux malades avaient un test positif dans la détection de l'antigène de surface de l'hépatite B (AgHBs), mais aussi un test négatif dans la détection de l'antigène AgHBe propre à l'hépatite B.

Ces résultats sérologiques excluaient que les virus de l'hépatite A ou de l'hépatite B soient la cause de l'épidémie dont les malades susdits étaient victimes. Il n'a pas non plus été trouvé d'anticorps spécifiques de la classe des IgM, anti-CMV, anti-virus Epstein-Barr ou anti-fièvre jaune.

## 2 - Préparation d'extraits de fèces

2.1. Des échantillons des selles de 10 malades (sur les 37 sujets) ont été prélevés les premiers jours après l'apparition des symptômes de la maladie. Ils ont servi à la préparation d'extraits de fèces à 10% dans du PBS contenant 1% de sérum-albumine humaine. Ces extraits de fèces ont été homogénéisés, puis clarifiés par centrifugation.

2.2. Les extraits de fèces clarifiés ont été filtrés à travers un filtre millipore 0,45 $\mu$m, puis à travers des filtres constitués par des disques à membrane de type HA de 0,22 $\mu$m, après quoi on les a fait réagir avec un volume égal de fractions IgM provenant de sérums de singes infectés, comme décrit en 2.3. ci-après, pendant 1 heure à 37°C, puis pendant une nuit à 4°C. Le mélange réactionnel est centrifugé pendant 20 minutes à 20000 g et le culot est mis en suspension dans du PBS. Un aliquot de cette préparation est dialysé contre de l'eau distillée, coloré négativement par de l'acide phosphotungstique à pH 7,2, puis examiné au microscope électronique SIEMENS.

2.3. Les extraits de fèces clarifiés ont été dilués dans l'eau à une concentration de 10% pour préparer un inoculum qui a été administré à raison de 2 ml par voie orale à 4 singes africains anesthésiés (3 Céropithécus aethiops et 1 Erythrocébus patas).

Des échantillons de sérums et de fèces de ces singes ont été collectés avant l'administration de l'inoculum et le 27ème jour après l'inoculation.

## 3 - Préparation d'IgM anti-NANBH

3.1. Les IgM anti-NANBH ont été isolées de sérums de singes infectés comme décrut en 2.3. ci-dessus, par chromatographie sur "SEPHADEX®G-200" (gel d'agarose fabriqué par PHARMACIA) dans du tampon Tris-NaCl 0,1 M, pH 8,0.

3.2. Les IgM anti-NANBH isolées ont été fixées sur des plaques en PVC (fournies par NUNC-IMMUNO-PLATES, DANEMARK) par contact de ces plaques pendant 24 heures à 4°C avec les IgM à une concentration de 50 $\mu$g de protéines/ml dans du PBS.

Les plaques ainsi traitées ont ensuite été revêtues de PBS contenant 0,1% de BSA et 0,1% de "Tween®20".

## 4 - Préparation d'une fraction d'IgG pour l'identification du système antigène-anticorps associé à la forme épidémique d'hépatite NANB Une fraction d'IgG a été obtenue par purification de sérums de patients convalescents de NANBH, par chromatographie sur colonne de DEAE-Trisacryl, puis les IgG purifiées obtenues ont été marquées par de la β-galactosidase.

5 - Test de diagnostic de la NANBH

5.1. Des IgM isolées de sérums de singes collectés préalablement à l'inoculation ont été utilisées pour enduire des plaques de PVC destinées à servir de témoins.

5.2. Des extraits de fèces de patients dont on cherche à établir le diagnostic d'hépatite NANB, préparés comme décrit en 2.1. ci-dessus (1:20 v/v) ont été incubés sur des plaques enduites d'IgM anti-NANBH, comme décrit ci-dessus (en 3.2.), pendant 1 heure à 37°C, puis avec une fraction d'IgG (200 μg/ml) marquées à la β-galactosidase, obtenues comme décrit en 4 ci-dessus, en présence d'orthonitrophényl-β-D-galactopyranoside comme substrat chromogène révélateur.

5.3. Les IgM spécifiques isolées des sérums de singes prélevés le 27ème jour après l'inoculation de ceux-ci, fixent de façon spécifique l'antigène présent dans les fèces de patients atteints l'hépatite NANB et l'antigène ainsi fixé est détecté par les IgG marquées par l'enzyme, comme décrit ci-dessus.

6 - Résultats Dans 5 sur 10 des extraits de fèces prélevés chez des malades présumés atteints de NANBH, comme décrit en 2.1. ci-dessus, l'antigène associé à la NANBH a pu être détecté, mais ne l'a été dans aucun des 30 extraits de fèces provenant de patients européens atteints d'autres infections gastro-intestinales (cf. Tableau ci-après). Tous les échantillons positifs lors du test immunoenzymatique utilisant des IgM isolées 27 jours après l'inoculation, avaient été trouvés négatifs dans le système témoin qui a utilisé des IgM isolées de sérums prélevés avant l'inoculation. L'agglutination de particules probablement virales a été observée au microscope électronique après réaction de l'anticorps IgM isolé de sérums de 4 patients, avec des extraits de fèces des mêmes patients.

Comme le montre le Tableau ci-dessous, il n'y a pas de rapport sérologique entre l'antigène de l'hépatite NANB épidémique présent dans les fèces de patients infectés et le virus de l'hépatite A ou l'antigène de l'hépatite B. En effect ce Tableau montre les résultats obtenus dans le test immunoenzymatique de détection de la présence d'antigène NANB dans des échantillons de fèces :

3 des 4 anticorps IgM isolés de sérums de singes infectés expérimentalement, fixés sur une phase solide (plaque de PVC) fixent de façon spécifique un antigène présent dans les-échantillons de fèces de patients présentant une forme épidémique d'hépatite NANB.

Deux fractions d'IgG isolées de sérums de patients convalescents d'une forme épidémique d'hépatite NANB sont été marquées à la β-galactosidase et utilisées comme révélateurs (* les résultats obtenus avec la 2ème fraction sont parallèles à ceux obtenus avec la 1ère fraction). Les résultats réunis dans le Tableau sont exprimés en rapport P/N (qui est le rapport absorption de l'échantillon testé/absorption de la moyenne des témoins négatifs). 6 extraits de fèces normales différents ont été utilisés comme témoins négatifs. Des résultats négatifs (P/N < 2,1) ont été obtenus pour des tests témoins effectués sur des plaques revêtues d'IgM isolées de sérums des singes préalablement à l'inoculation de ces derniers.

## TABLEAU

### DOSAGE IMMUNOENZYMATIQUE DE L'ANTIGENE ASSOCIE A UNE HEPATITE NANB EPIDEMIQUE

| Echantillon de fèces | Singe n° 1 | Singe n° 2 | Singe n° 3 |
|---|---|---|---|
| 1 | 2,18 | 4,98<br>6,20 | 2,1 |
| 2 | 2,1 | 6,37<br>4,25* | 3,13<br>3,19 |
| 3 | 18,5* | 4,37<br>4,66 | 3,36 |
| 4 | 6,06<br>17,7* | 2,35<br>3,25* | 2,1 |
| 5 | 2,1 | 2,1 | non dosé(n.d.) |
| 6 | 6,39 | 5,20 | 6,22 |
| 7 | 2,1 | n.d. | 2,1 |
| 8 | 2,17 | 2,1 | n.d. |
| 9 | 2,1 | 2,1 | n.d. |
| 10 | 2,1 | 2,1 | n.d. |

* Voir Section 6 "Résultats" page 10.

L'identification du virus d'hépatite NANB épidémique sur laquelle se fonde la présente invention a mis en évidence que l'agent étiologique présumé de cette forme d'hépatite n'a de lien ni avec le virus de l'hépatite A ni avec le virus de l'hépatite B et qu'il est distinct des deux types d'hépatite NANB transmis par des transfusions sanguines.

Conformément à l'invention, des IgM isolées de sérums de mammifères infectés permettent de réaliser des tests de diagnostic de l'hépatite NANB par caractérisation immunoenzymatique d'un antigène associé au virus de NANBH présent dans les fèces de patients atteints d'une forme épidémique d'hépatite NANB.

## Revendications
## Revendications pour les Etats contractants suivants : BE, CH, DE, FR, GB, IT, LI, LU, NL, SE

1. Anticorps anti-NANBH constitué par des IgM isolées de sérums de singes infectés artificiellement par des extraits de fèces de patients connus pour être atteints d'hépatite NANB épidémique.

2. Procédé d'isolement d'un anticorps anti-NANBH selon la revendication 1 à partir de sérums de singes artificiellement infectés par ingestion d'extraits de fèces de patients atteints d'hépatite NANB épidémique, caractérisé en ce que lesdits sérums sont traités par chromatographie sur colonne de gel de dextrane dans du tampon Tris-NaCl 0,1 M, pH 8,0.

3. Procédé selon la revendication 2, caractérisé en ce que les sérums de singes artificiellement infectés mis en oeuvre sont des sérums prélevés à partir du 27ème jour après l'inoculation.

4. Réactif propre à être utilisé pour la détection d'hépatites virales NANB épidémiques, caractérisé en ce qu'il comprend des IgM anti-NANBH selon la revendication 1 fixées sur des supports solides.

5. Procédé de préparation d'un réactif selon la revendication 4, caractérisé en ce que les IgM isolées conformément à l'une quelconque des revendications 2 ou 3, sont fixées sur des supports solides et notamment sur des plaques en PVC, par contact des IgM à des concentrations de l'ordre de 50 $\mu$g de protéines par ml de PBS, avec lesdits supports solides pendant 18 à 24 heures à 4° C.

6. Procédé selon la revendication 5, caractérisé en ce que les supports solides tels que plaques de PVC notamment, revêtus d'IgM anti-NANBH, sont ensuite enduits d'une couche de PBS contenant 0,1% de BSA et 0,1% de "Tween®20".

7. Méthode de diagnostic d'hépatites virales NANB épidémiques, caractérisée en ce que le réactif de détection selon la revendication 4 est incubé avec des extraits de fèces pendant 1 heure à 37° C, puis avec des fractions d'IgG purifiées à partir de sérums de patients convalescents de NANBH épidémique et marquées par une enzyme, en présence d'un substrat révélateur approprié.

8. Méthode de diagnostic selon la revendication 7, caractérisée en ce que les fractions d'IgG sont marquées à la $\beta$-galactosidase et le substrat révélateur approprié est de préférence constitué par l'orthonitrophényl-$\beta$-D-galacto-pyranoside.

9. Kit de diagnostic d'hépatites virales NANB épidémiques, caractérisé en ce qu'il comprend, outre les tampons, solvants et réactifs usuels nécessaires à la mise en oeuvre de la méthode de diagnostic selon l'une quelconque des revendications 7 et 8, au moins un réactif de diagnostic selon la revendication 4, au moins un réactif constitué par des IgG purifiées à partir de sérums de patients convalescents de NANBH épidémique et marquées par une enzyme, et au moins un substrat révélateur de la réaction enzymatique, approprie.

## Revendications pour les Etats contractants suivants : AT, ES, GR

1. Procédé d'isolement d'un anticorps anti-NANBH caractérisé en ce que ledit anticorps est constitué par des IgM isolées de sérums de singes infectés artificiellement par des extraits de fèces de patients connus pour être atteints d'hépatite NANB épidémique.

2. Procédé d'isolement d'un anticorps anti-NANBH selon la revendication 1 à partir de sérums de singes artificiellement infectés par ingestion d'extraits de fèces de patients atteints d'hépatite NANB épidémique, caractérisé en ce que lesdits sérums sont traités par chromatographie sur colonne de gel de dextrane dans du tampon Tris-NaCl 0,1 M, pH 8,0.

3. Procédé selon la revendication 2, caractérisé en ce que les sérums de singes artificiellement infectés

EP 0 242 300 B1

mis en oeuvre sont des sérums prélevés à partir du 27ème jour après l'inoculation.

4. Procédé de préparation d'un réactif propre à être utilisé pour la détection d'hépatites virales NANB épidémiques, caractérisé en ce que ledit réactif comprend des IgM anti-NANBH préparées par le procédé selon la revendication 1 fixées sur des supports solides.

5. Procédé de préparation d'un réactif selon la revendication 4, caractérisé en ce que les IgM isolées conformément à l'une quelconque des revendications 2 ou 3, sont fixées sur des supports solides et notamment sur des plaques en PVC, par contact des IgM à des concentrations de l'ordre de 50 $\mu$g de protéines par ml de PBS, avec lesdits supports solides pendant 18 à 24 heures à 4°C.

6. Procédé selon la revendication 5, caractérisé en ce que les supports solides tels que plaques de PVC notamment, revêtus d'IgM anti-NANBH, sont ensuite enduits d'une couche de PBS contenant 0,1% de BSA et 0,1% de "Tween®20".

7. Procédé de diagnostic d'hépatites virales NANB épidémiques, caractérisé en ce que ledit procédé consiste à incuber le réactif de détection préparé par le procédé selon la revendication 4 avec des extraits de fèces pendant 1 heure à 37°C, puis avec des fractions d'IgG purifiées à partir de sérums de patients convalescents de NANBH épidémique et marquées par une enzyme, en présence d'un substrat révélateur approprié.

8. Procédé selon la revendication 7, caractérisé en ce que les fractions d'IgG sont marquées à la $\beta$-galactosidase et le substrat révélateur approprié est de préférence constitué par l'orthonitrophényl-$\beta$-D-galacto-pyranoside.

9. Système de diagnostic, caractérisé en ce que ledit système comprend, outre les tampons, solvants et réactifs usuels nécessaires à la mise en oeuvre du procédé de diagnostic selon l'une quelconque des revendications 7 et 8, au moins un réactif de diagnostic préparé par le procédé selon la revendication 4, au moins un réactif constitué par des IgG purifiées à partir de sérums de patients convalescents de NANBH épidémique et marquées par une enzyme, et au moins un substrat révélateur de la réaction enzymatique, approprié.

**Claims**
**CLAIMS for the following Contracting States : BE, CH, DE, FR, GB, IT, LI, LU, NL, SE**

1. An anti-NANBH antibody consisting of IgM's isolated from the sera of monkeys artificially infected with extracts of faeces from patients known to be suffering from epidemic NANB hepatitis.

2. A method of isolating an anti-NANBH antibody according to Claim 1 from the sera of monkeys artificially infected by the ingestion of extracts of faeces from patients suffering from epidemic NANB hepatitis, characterized in that said sera are treated by chromatography on a column of dextran gel in Tris-NaCl 0.1 M buffer, pH 8.0.

3. A method according to Claim 2, characterized in that the sera of artificially infected monkeys used are sera taken as from the 27th day after inoculation.

4. A reagent suitable for use in the detection of epidemic NANB viral hepatites, characterized in that it comprises anti-NANBH IgM's according to Claim 1, bound to solid supports.

5. A method of preparing a reagent according to Claim 4, characterized in that the IgM's isolated according to Claim 2 or Claim 3 are bound to solid supports, and especially to PVC plates, by bringing the IgM's, at concentrations of the order of 50 $\mu$g of proteins per ml of PBS, into contact with said solid supports for 18 to 24 hours at 4°C.

6. A method according to Claim 5, characterized in that the solid supports such as PVC plates in particular, coated with anti-NANBH IgM's, are then coated with a layer of PBS containing 0.1% of BSA and 0.1% of "Tween® 20".

8

7. A method of diagnosing epidemic NANB viral hepatites, characterized in that the detection reagent according to Claim 4 is incubated with faeces extracts for 1 hour at 37° C and then with IgG fractions purified from the sera of patients convalescing from epidemic NANBH, and labelled with an enzyme, in the presence of an appropriate developing substrate.

8. A diagnostic method according to Claim 7, characterized in that the IgG fractions are labelled with $\beta$-galactosidase and the appropriate developing substrate preferably consists of orthonitrophenyl-$\beta$-D-galacto-pyranoside.

9. A kit for diagnosing epidemic NANB viral hepatites, characterized in that it comprises, in addition to the customary buffers, solvents and reagents required for carrying out the diagnostic method according to Claim 7 or Claim 8, at least one diagnostic reagent according to Claim 4, at least one reagent consisting of IgG's purified from the sera of patients convalescing from epidemic NANBH, and labelled with an enzyme, and at least one appropriate substrate for developing the enzymatic reaction.

**CLAIMS for the following Contracting States : AT, ES, GR**

1. A method of isolating an anti-NANBH antibody, characterized in that said antibody consists of IgM's isolated from the sera of monkeys artificially infected with extracts of faeces from patients known to be suffering from epidemic NANB hepatitis.

2. A method, according to Claim 1, of isolating an anti-NANBH antibody from the sera of monkeys artificially infected by the ingestion of extracts of faeces from patients suffering from epidemic NANB hepatitis, characterized in that said sera are treated by chromatography on a column of dextran gel in Tris-NaCl 0.1 M buffer, pH 8.0.

3. A method according to Claim 2, characterized in that the sera of artificially infected monkeys used are sera taken as from the 27th day after inoculation.

4. A method of preparing a reagent suitable for use in the detection of epidemic NANB viral hepatites, characterized in that said reagent comprises anti-NANBH IgM's prepared by the method according to Claim 1 and bound to solid supports.

5. A method of preparing a reagent, according to Claim 4, characterized in that the IgM's isolated according to Claim 2 or Claim 3 are bound to solid supports, and especially to PVC plates, by bringing the IgM's, at concentrations of the order of 50 $\mu$g of proteins per ml of PBS, into contact with said solid supports for 18 to 24 hours at 4° C.

6. A method according to Claim 5, characterized in that the solid supports such as PVC plates in particular, coated with anti-NANBH IgM's, are then coated with a layer of PBS containing 0.1% of BSA and 0.1% of "Tween® 20".

7. A method of diagnosing epidemic NANB viral hepatites, characterized in that said method consists in incubating the detection reagent prepared by the method according to Claim 4 with faeces extracts for 1 hour at 37° C, and then with IgG fractions purified from the sera of patients convalescing from epidemic NANBH, and labelled with an enzyme, in the presence of an appropriate developing substrate.

8. A method according to Claim 7, characterized in that the IgG fractions are labelled with $\beta$-galactosidase and the appropriate developing substrate preferably consists of orthonitrophenyl-$\beta$-D-galactopyranoside.

9. A diagnostic system, characterized in that said system comprises, in addition to the customary buffers, solvents and reagents required for carrying out the diagnostic method according to Claim 7 or Claim 6, at least one diagnostic reagent prepared by the method according to Claim 4, at least one reagent consisting of IgG's purified from the sera of patients convalescing from epidemic NANBH, and labelled with an enzyme, and at least one appropriate substrate for developing the enzymatic reaction.

**Patentansprüche**

**Patentansprüche für folgende Vertragsstaaten : BE, CH, DE, FR, GB, IT, LI, LU, NL, SE**

1. NANB-Hepatitis-Antikörper, der durch IgM gebildet wird, welche aus Sera von Affen isoliert werden, die künstlich mit Fäkalienextrakten nachgewiesener NANB-Hepatitis-Patienten infiziert wurden, damit sie von epidemischer NANB-Hepatitis befallen werden.

2. Verfahren zur Isolierung eines NANB-Hepatitis-Antikörpers nach Anspruch 1, ausgehend von Sera von Affen, die durch Einnahme von Fäkalienextrakten von von epidemischer NANB-Hepatitis befallenen Patienten künstlich infiziert wurden, dadurch gekennzeichnet, daß die Sera mittels Dextrangel-Säulenchromatographie in einem Tris-NaCl 0,1 M, pH 8,0 Puffer behandelt werden.

3. Verfahren nach Anspruch 2, dadurch gekennzeichnet, daß die verwendeten Sera der künstlich infizierten Affen Sera sind, die ab dem 27 Tag nach der Inokulation entnommen werden.

4. Reagenz geeignet für den Nachweis von epidemischer NANB-Virushepatitis, dadurch gekennzeichnet, daß es Anti-NANB-IgM gemäß Anspruch 1 umfaßt, die auf festen Trägern fixiert sind.

5. Verfahren zur Herstellung eines Reagenz nach Anspruch 4, dadurch gekennzeichnet, daß die gemäß einem der Ansprüche 2 oder 3 isolierten IgM auf festen Trägern, insbesondere auf PVC-Platten durch Kontakt der IgM, die Konzentrationen in der Größenordnung von 50 $\mu$g Proteinen je PBS-ml aufweisen, mit den festen Trägern während 18 bis 24 Stunden bei 4°C fixiert sind.

6. Verfahren nach Anspruch 5, dadurch gekennzeichnet, daß die festen mit Anti-NANB-IgM überzogenen Träger, wie etwa insbesondere die PVC-Platten, in der Folge mit einer PBS-Schicht überzogen werden, die 0,1% BSA und 0,1% "Tween ® 20" enthält.

7. Verfahren zur Diagnose von epidemischer NANB-Virushepatitis, dadurch gekennzeichnet, daß das Reagenz zum Nachweis nach Anspruch 4 mit Fäkalienextrakten während 1 Stunde bei 37°C, danach mit von Sera von nach einer epidemischen NANB-Hepatitis kovaleszenten Patienten stammenden gereinigten IgG-Fraktionen inkubiert wird und in Anwesenheit eines entsprechenden Nachweissubstrats mit einem Enzym markiert wird.

8. Verfahren zur Diagnose nach Anspruch 7, dadurch gekennzeichnet, daß die IgG-Fraktionen mit $\beta$-Galactosidase markiert sind, und das entsprechende Nachweissubstrat vorzugsweise durch Orthonitrophenyl-$\beta$–D-Galactopyranosid gebildet wird.

9. Set zur Diagnose von epidemischer NANB-Virushepatitis, dadurch gekennzeichnet, daß es außer den zur Durchführung des Diagnoseyerfahrens nach einem der Ansprüche 7 und 8 notwendigen Puffern, Lösungsmitteln und herkömmlichen Reagenzien mindestens ein Diagnosereagenz nach Anspruch 4, mindestens ein durch Sera von nach einer epidemischen NANB-Hepatitis kovaleszenten Patienten stammenden, gereinigten und mit einem Enzym markierten IgG gebildetes Reagenz und mindestens ein entsprechendes Nachweissubstrat für die enzymatische Reaktion beinhaltet.

**Patentansprüche für folgende Vertragsstaaten : AT, ES, GR**

1. Verfahren zur Isolierung eines NANB-Hepatitis-Antikörpers, dadurch gekennzeichnet, daß der Antikörper durch IgM gebildet wird, welche aus Sera von Affen isoliert werden, die künstlich mit Fäkalienextrakten nachgewiesener NANB-Hepatitis-Patienten infiziert wurden, damit sie von epidemischer NANB-Hepatitis befallen werden.

2. Verfahren zur Isolierung eines NANB-Hepatitis-Antikörpers nach Anspruch 1, ausgehend von Sera von Affen, die durch Einnahme von Fäkalienextrakten von von epidemischer NANB-Hepatitis befallenen Patienten künstlich infiziert wurden, dadurch gekennzeichnet, daß die Sera mittels Dextrangel-Säulenchromatographie in einem Tris-NaCl 0,1 M, pH 8,0 Puffer behandelt werden.

3. Verfahren nach Anspruch 2, dadurch gekennzeichnet, daß die verwendeten Sera der künstlich infizierten Affen Sera sind, die ab dem 27 Tag nach der Inokulation entnommen werden.

4. Verfahren zur Herstellung eines Reagenzs, das für den Nachweis von epidemischer NANB-Virushepatitis geeignet ist, dadurch gekennzeichnet, daß das Reagenz nach dem Verfahren gemäß Anspruch 1 hergestellte Anti-NANB-IgM umfaßt, die auf festen Trägern fixiert sind.

5. Verfahren zur Herstellung eines Reagenz nach Anspruch 4, dadurch gekennzeichnet, daß die gemäß einem der Ansprüche 2 oder 3 isolierten IgM auf festen Trägern, insbesondere auf PVC-Platten durch Kontakt der IgM, die Konzentrationen in der Größenordnung von 50 μg Proteinen je PBS-ml aufweisen, mit den festen Trägern während 18 bis 24 Stunden bei 4° C fixiert sind.

6. Verfahren nach Anspruch 5, dadurch gekennzeichnet, daß die festen mit Anti-NANB-IgM überzogenen Träger, wie etwa insbesondere die PVC-Platten, in der Folge mit einer PBS-Schicht überzogen werden, die 0,1% BSA und 0,1% "Tween ® 20" enthält.

7. Verfahren zur Diagnose von epidemischer NANB-Virushepatitis, dadurch gekennzeichnet, daß das Verfahren darin besteht, das nach dem Verfahren gemäß Anspruch 4 hergestellte Nachweisreagenz während 1 Stunde bei 37° C, danach mit von Sera von nach einer epidemischen NANB-Hepatitis kovaleszenten Patienten stammenden gereinigten IgG-Fraktionen inkubiert wird und in Anwesenheit eines entsprechenden Nachweissubstrats mit einem Enzym markiert wird.

8. Verfahren nach Anspruch 7, dadurch gekennzeichnet, daß die IgG-Fraktionen mit β-Galactosidase markiert sind, und das entsprechende Nachweissubstrat vorzugsweise durch Orthonitrophenyl-β-D-Galactopyranosid gebildet wird.

9. Diagnosesystem, dadurch gekennzeichnet, daß es außer den zur Durchführung des Diagnoseverfahrens nach einem der Ansprüche 7 und 8 notwendigen Puffern, Lösungsmitteln und herkömmlichen Reagenzien mindestens ein Diagnosereagenz nach Anspruch 4, mindestens ein durch Sera von nach einer epidemischen NANB-Hepatitis kovaleszenten Patienten stammenden, gereinigten und mit einem Enzym markierten IgG gebildetes Reagenz und mindestens ein entsprechendes Nachweissubstrat für die enzymatische Reaktion beinhaltet.